# EUROPEAN PATENT APPLICATION

(11) **EP 2 851 065 A1**
(43) Date of publication of application: **25.03.2015**
(21) Application number: 14185170.9
(22) Date of filing: 16.01.2009
(51) Int. Cl.: A61K 9/08, A61K 33/04, A61K 47/02, A61P 13/04, A61P 13/10, A61P 13/12

(54) **Sulfate salt prophylaxis**

(30) Priority: 16.01.2008 US 11347
(62) Divisional of application: 09701859.2
(71) Applicant: Braintree Laboratories, Inc., Braintree, MA 02185-0929 (US)
(72) Inventor: Fordtran, John, S., Dallas, TX Texas 75256 (US)
(74) Representative: Grund, Martin

(57) **Abstract**

Sulafte salts are used to inhibit the development of, or to treat, hypercalcium concentration-related disorders.

## Description

### FUNDING

This study was supported by the Southwest Digestive Disease Foundation and Braintree Laboratories.

### BACKGROUND OF THE INVENTION

Phosphate catharsis can cause renal tubular deposition of calcium phosphate and renal failure. Existing methods to cleanse the colon prior to colonoscopy include: (1) oral ingestion of four liters of solution containing polyethylene glycol (PEG 3350); or (2) oral ingestion of two 45 mL doses of concentrated sodium phosphate solution (given 10 hours apart), with additional water to mitigate dehydration. Most patients prefer and are more compliant with the small volume phosphate solution (Barkun, et al. (2006) Can. J. Gastroenterol. 20:699-710). However, phosphate catharsis occasionally causes kidney failure characterized by calcium phosphate deposition in distal tubules (Desmeules, et al. (2003) N. Eng. J. Med. 349:1006-1007; Markowitz, et al. (2004) Hum..Pathol. 35:675-684; Markowitz, et al. (2005) J. Amer. Soc. Nephrol. 16:3389-3396; Gonlusen, et al. (2006) Arch. Pathol. Lab. Med. 130:101-106), a complication most likely mediated by high concentrations of phosphate in renal tubular fluid. The recent recognition of this form of phosphate induced kidney disease has created a dilemma for patients and physicians, i.e., a choice between a small risk of renal failure with phosphate cathartics versus risk of poor compliance with large volume PEG solution.

A possible resolution of this dilemma is to develop a small volume phosphate-free cathartic that would be as effective as phosphate, but less likely to cause kidney damage. However, no experimental studies have been conducted to assess the relative propensity of orally ingested sulfate and phosphate to induce precipitation of calcium salts within the kidneys. Like phosphate, sulfate is actively absorbed by the intestine (Morris, et al. (2001) J. Membrane Biol. 181:1-9), and from concentrated solutions passive sulfate absorption might be substantial. Moreover, in laboratory animals intravenous infusion of sodium sulfate is associated with increased urine calcium excretion (Walser, et al. (1959) J. Clin. Invest. 38:1404-1411). If substantial amounts of sulfate were absorbed after oral ingestion of a concentrated sulfate solution, and if this produced hypercalciuria, then the risk of calcium precipitation in renal tubular fluid might increase.

### SUMMARY

The present inventors have discovered that the treatment of subjects with sodium, potassium and magnesium sulfates can inhibit the formation of, and treats, calcium stones or precipitations in subjects with hypercalcium concentration-related disorders. This discovery has been exploited to develop the present invention which is at least in part related to the following.

In a first aspect, the disclosure provides a method of reducing the risk of renal calcinosis occurring from the ingestion of a lavage solution in a patient with a propensity for renal stones. The method comprises administering a lavage solution consisting of sodium sulfate, potassium sulfate, and magnesium sulfate in the patient. In some embodiments, the lavage solution contains between about 5 g to about 30 g of sodium sulfate, between about 1 g to about 5 g potassium sulfate, and between about 0.5 g to about 5 g of magnesium sulfate In other embodiments, the volume of the lavage solution is between about 75 ml and 125 ml. In still further embodiments, there is a first administration of the lavage solution and a second administration of the lavage solution. In other embodiments, the lavage solution contains about 17 g of sodium sulfate, about 3 grams of potassium sulfate, and about 1.5 g of magnesium sulfate. In other embodiments, the administration of the lavage solution is immediately followed by the administration of water. In other embodiments, the volume of the water immediately following the lavage solution is between about 200 ml and 300 ml. In other embodiments, the method further consists of additional administration of water after the volume of 200 to 300 ml of water after administration of the lavage solution. In still further embodiments, the additional administration of water consists of a volume of between about 1200 ml and about 2000 ml. In particular embodiments, the volume of water is about 1600 ml. In some embodiments, the additional administration of water after the administration of the lavage solution is divided into equal doses every 30 minutes for three hours.

In another aspect, the disclosure provides a method of treating a hypercalcium concentration-related disorder in a patient in need thereof. The method comprises the administration of a composition comprising sodium sulfate, potassium sulfate, and magnesium sulfate. In one embodiment, the composition comprises sodium sulfate, potassium sulfate, and magnesium sulfate. In another embodiment, the composition is in the form of a solution. In some embodiments, the disorder is selected from the group consisting of hypercalciuria, nephrocalcinosis, recurring calcium nephrolithiasis, calcium phosphate stone disease, stone forming disease, struvite formation, calcium-phosphate recurrent stones, absorptive hypercalciuria syndrome, vascular calcification, recurrent calcium urolithiasis, persistently high urinary pH, calciphylaxis, calciphalaxis, chronic renal failure, end stage renal disease, calcific uremic arteriolopathy.

In yet another aspect, the use of a solution of sodium sulfate, potassium sulfate, and magnesium sulfate in the manufacture of a medicament for reducing the risk of renal calcinosis occurring from the ingestion of a lavage solution in a patient with a propensity for renal stones is provided. The medicament comprises the solution containing sodium sulfate, potassium sulfate, and magnesium sulfate which is administered to the patient. In a different aspect, the use of sodium sulfate, potassium sulfate, and magnesium sulfate in the manufacture of a medicament for the treatment a hypercalcium concentration-related disorder in a patient in need thereof, is provided.

### BRIEF DESCRIPTION OF THE DRAWINGS

Figure 1A is a graphic representation of the percent absorption of phosphate and sulfate from orally ingested cathartic solutions.
Figure 1B is a graphic representation of the correlation between percent phosphate absorption and the concentration of phosphate in serum obtained three hours after ingestion of the second dose of phosphate solution.
Figure 1C is a graphic representation of the correlation between percent phosphate absorption and urine phosphate excretion during the 17 hour experimental period.
Figure 2A is a graphic representation of the correlation between cumulative stool weight and percent insoluble fecal matter in individual subjects, where the results after the first dose are shown by open symbols; results after both doses are shown by closed symbols.
Figure 2B is a photographic representation of diarrheal stool cleansing, for comparison with results shown in Figure 2A, where liters of diarrhea at the bottom of the figure refer to cumulative stool weight when specimens were collected for centrifugation, and the percent of diarrheal stool composed of insoluble fecal matter has been calculated for each specimen.

### DESCRIPTION OF THE INVENTION

As used herein, a subject or patient "in need thereof" is one suffering, or having the propensity to suffer from, a hypercalcium concentration-related disorder.

One purpose of these studies is to compare the intestinal and renal effects of phosphate catharsis versus sulfate catharsis. A large volume PEG based lavage solution was used as a control. Sequential doses (10 hours apart) of each cathartic/lavage solution were administered to normal subjects. Considering recent safety concerns, the second dose of phosphate was reduced from 45 to 30 mL. This resulted in nearly identical total molar doses of phosphate and sulfate.

### A. Studies on Diarrheal Fluid

Although the doses of sulfate and phosphate (in mmoles) were almost identical, sulfate produced 43% more diarrheal stool volume. At least two factors contributed to the greater cathartic effect of sulfate. First, phosphate absorption was twice that of sulfate absorption. Higher absorption of phosphate reduces the quantity of water obligating osmoles in stool, and thereby reduces stool volume. Second, while the valence of sulfate is 2, the valence of phosphate varies with pH and within intestinal fluid is generally less than 2. Therefore, one mmole of unabsorbed phosphate would obligate fewer mmoles of unabsorbed cations and a lower volume of intraluminal fluid than one mmole of unabsorbed sulfate.

Cleansing of diarrheal fluid of insoluble fecal matter was highly correlated with the volume of diarrhea produced by the three cathartic/lavage solutions. Presumably because sulfate induced more diarrhea than phosphate, the sulfate cathartic provided better cleansing of diarrheal fluid than the phosphate cathartic.

In preliminary studies to develop a single dose of the concentrated sulfate solution that was employed here, the relative quantities of sodium sulfate, potassium sulfate and magnesium sulfate were adjusted until a mixture that did not cause clinically significant net absorption or net secretion of sodium, potassium or magnesium was achieved. In the present study, two doses of this sulfate solution resulted in minimal net cation absorption or secretion. The gastrointestinal effects of this blend and dose of sulfate salts, when accompanied by water as provided in this experiment, will not generate disorders of potassium, magnesium or sodium balance.

### B. Studies on Urine after Ingestion of Phosphate

Phosphate catharsis caused three major changes in urine composition: 1) a marked increase in phosphate concentration; 2) a marked fall in calcium concentration; and 3) a fall in pH. Although the low calcium concentration and low pH of urine after phosphate ingestion prevented precipitation of calcium phosphate in excreted urine, it is important to recognize that the calcium concentration and pH of excreted urine do not reflect conditions in renal tubular fluid, and that the relative saturation ratio for brushite (RSR-Br) in tubular fluid may be much higher than that in excreted urine (Asplin, et al. (1996) Am. J. Physiol. 270:F604-F613). To estimate the propensity of renal tubular fluid to form a calcium salt *precipitate, in vitro* experiments were carried out at the presumed pH of fluid within the loop of Henle and early distal tubule. In the excreted urine following phosphate ingestion, calcium phosphate precipitated at very low calcium concentrations at both pH 7.2 and pH 6.4, indicating a marked propensity of these urines to precipitate. In contrast, after PEG or sulfate, much higher calcium concentrations were used to initiate precipitation. This dramatic difference is presumably due mainly to the very high phosphate concentration in urine following ingestion of phosphate.

Normal tubular fluid at the bend of the loop of Henle has a pH of about 7.4, a calcium concentration of about 14 mg/dL, and a phosphate concentration of about 4.5 mg/dL (Fine et al. (1998) Dig. Dis. Sciences 43:2708-2714). To estimate the phosphorus concentration at the loop of Henle in the subjects, the following assumptions were made: 1) The quantity of phosphate excreted in final urine per unit time is equal to the quantity of phosphate delivered to the loop of Henle per unit time (i.e., phosphorus reabsorption takes place only in the proximal tubule); and 2) The volume of fluid delivered to the loop of Henle per unit time is equal to 40% of the GFR (i.e., proximal tubule volume reabsorption is 60%). On this basis, the phosphate concentration at the loop of Henle was 5.9 mg/dL prior to the first dose of phosphate and 17.4 mg/dL after the second dose. It was assumed that the pH and calcium concentration in this segment would remain unchanged, at pH 7.4 and 14 mg/dL, respectively (Fine, *et al.* ibid.). The RSR-Br of such tubular fluid would therefore increase dramatically after ingestion of phosphate, and thus the fluid would be highly prone to develop a brushite precipitate. In normal people who ingest a phosphate cathartic, the combination of augmented distal tubular acid secretion and calcium reabsorption would cause pH and calcium concentration to fall, and thus would limit downstream calcium phosphate precipitation. However, some people who ingest a phosphate cathartic will have underlying abnormalities in tubular calcium absorption and/or tubular acid secretion and these could cause pH and calcium concentrations in distal tubular fluid to remain high. Thus, calcium phosphate crystals that might form in the loop of Henle could travel downstream to the distal tubule and either dissolve under normal conditions, or act as nucleating sites and promote massive crystallization if acidification or calcium reabsorption is compromised. Potential underlying abnormalities in tubular calcium absorption include idiopathic or genetic calcium transport disorders, and loop diuretics. Abnormalities in tubular acid secretion include renal tubular acidosis and ingestion of carbonic anhydrase inhibitors. Other factors that accentuate calcium phosphate deposition include: 1) hyperabsorption of the phosphate load (vitamin D supplements, or reduced intestinal motility due to neuromuscular disease or opiate drugs); 2) hypercalciuric conditions; and 3) reduction in urine precipitation inhibitors, such as citrate (systemic acid loads, hypokalemia). Several of these abnormalities can exist in clinically silent forms, and thus be unrecognized prior to colonoscopy.

In the present study the subjects who ingested the concentrated phosphate solution were vigorously hydrated, and in spite of approximately 2 L of diarrhea they had a net absorption of over 1 liter of water, and they had urine volumes that were almost as high as diarrheal stool volumes. Such hydration has several benefits, including: 1) mitigation of an acute fall in GFR; 2) reduction of the stimulus (ECF volume contraction) for increased proximal tubular volume reabsorption, thereby mitigating a rise in the concentration of precipitating solutes in fluid delivered to the loop of Henle; and 3) decreased water absorption in the late distal tubule and collecting ducts, thereby decreasing the concentration of precipitating solutes in these segments of the nephron (Jamison, et al. (1992) Handbook of Physiology, Section 8. Renal Physiology, Volume II. Edited by Windhager EE. Oxford University Press, New York, 1992:1219-1280). Extrapolations suggest that subjects in this study had an increased risk of developing brushite precipitation at the loop of Henle and that without normal distal tubular acid secretion and calcium absorption that these subjects would be at increased risk for precipitation of calcium phosphate in their distal tubules. Consequently, aggressive hydration alone may not always prevent renal failure in susceptible people who ingest cathartic doses of phosphate.

Retrospective case studies of phosphate induced kidney failure have emphasized several risk factors, including advanced age, female sex, hypertension, preexisting renal disease, and drugs that reduce renal blood flow (ACE inhibitors and NSAIDS).

### C. Studies on Urine After Ingestion of Concentrated Sulfate Solution

Oral ingestion of sulfate caused an increase in the serum concentration of sulfate, but unlike previous studies where large doses of sulfate were infused intravenously (Chakmakjian, et al. (1966) N. Eng. J. Med. 75:862-869) serum calcium concentration did not fall and urine calcium excretion did not increase. There was, therefore, no increased propensity of urine to precipitate on account of hypercalciuria.

As urine calcium concentration was increased to induce precipitation at pH 6.4 or pH 7.2, the precipitate that formed was, with one exception, calcium phosphate rather than calcium sulfate, even though the urine phosphate concentration was normal and the sulfate concentration of urine was high. This marked predilection for calcium phosphate precipitation exists because calcium sulfate is much more soluble than calcium phosphate. For example, using standard equilibrium constants, the relative solubility of CaSO₄ to CaHPO₄ is 2.9 at pH 5.5, 6.3 at pH 6.4, and 8.6 at pH 7.2.

Compared to urine collected prior to ingestion of sulfate, urine produced after sulfate ingestion had a much higher calcium concentration to induce precipitation at pH 6.4, and the difference was highly statistically significant.
that decrease ionized calcium concentration. However, calculations based on published values for equilibrium constants reveal that sulfate-calcium complexation cannot account for a substantial fraction of the observed sulfate effect at pH 6.4. It may be that sulfate production in the urine of a species that complexes with calcium and works synergistically with sulfate to sequester ionized calcium, or a direct inhibitory effect of sulfate on calcium phosphate crystallization, is involved. At pH 7.2 the effect of sulfate was smaller and not statistically significant. This can be explained, at least in part, by the fact that at pH 7.2 there is more phosphate in the form of HPO₄⁻² (the principal precipitating moiety), thereby increasing the ability of phosphate to compete with sulfate or sulfate surrogates for ionized calcium. Regardless of the mechanisms involved, the sulfate induced resistance to precipitation of calcium at pH 6.4 may be particularly important in the matter at hand, since this is the expected pH in the early distal tubule.

On the basis of this research, sulfate is a more effective cathartic than phosphate, and low volume concentrated sulfate catharsis would be less likely than low volume concentrated phosphate catharsis to induce calcium salt deposition in renal tubular fluid. Generous hydration should remain an integral part of a sulfate cathartic regimen, to prevent vascular instability from dehydration and to further reduce the likelihood of crystallization in renal tubular fluid. Insuring such hydration is a challenge, especially when the second dose is ingested during normal sleeping hours.

### D. Therapies

The invention provides for treatment or prevention of hypercalcium concentration-related disorders in subjects such as mammals and more particularly, humans. The treatment and/or prevention of hypercalcium concentration-related disorders includes, but is not limited to, alleviating symptoms associated with such disorders, the inhibition of the progression of such disorders, and the promotion of the regression of such disorders. It is contemplated that the dose of total sulfate derived magnesium sulfate, sodium sulfate, and potassium sulfate in the therapeutic solution, is between about 0.01 mg/ml to about 100 mg/ml, or 0.1 mg/ml to about 10 mg/ml, or 1 mg/ml to about 5 mg/ml.

The sulfate therapeutic treatment described herein can be administered in combination with other types of known treatments for these disorders . Such other treatments can be administered to a patient for the prevention or treatment of hypercalcium concentration-related disorders prior to *(e.g.,* 1 min., 15 min., 30 min., 45 min., 1 hour, 2 hours, 4 hours, 6 hours, 8 hours, 12 hours, 24 hours, 2 days, or 1 week before), subsequent to *(e.g.,* 1 min., 15 min., 30 min., 45 min., 1 hour, 2 hours, 4 hours, 6 hours, 8 hours, 12 hours, 24 hours, 2 days, or 1 week after), or concomitantly with, the administration of the therapeutics according to this disclosure to the subject.

### E. Pharmaceutical Formulations and Methods of Treatment

The present invention provides for both prophylactic and therapeutic methods of treating a subject having a hypercalcium concentration-related disorder. Administration of a prophylactic treatment according to this disclosure can occur prior to the manifestation of symptoms characteristic of the hypercalcium concentration-related disorders. For such therapy, the therapeutic composition according to the disclosure can be formulated for a variety of loads of administration, including oral, anal, systemic, or localized administration. Techniques and formulations generally may be found in Remmington's Pharmaceutical Sciences, Meade Publishing Co., Easton, Pa.

For example, liquid preparations for oral administration may take the form of lavages, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); and preservatives (e.g., methyl or propyl-p-hydroxybenzoates or sorbic acid). The preparations may also contain flavoring, coloring and sweetening agents as appropriate.

### EXAMPLES

Those skilled in the art will recognize, or be able to ascertain, using no more than routine experimentation, numerous equivalents to the specific substances and procedures described herein. Such equivalents are intended to be encompassed in the scope of the claims that follow the examples below.

### 1. Methods of

### A. Subjects

Well hydrated normal subjects ingested 75 ml of phospho-soda or an equimolar dose of sulfate salts. PEG lavage solution was the control. Propensity of urine to precipitate at a specified pH was assessed by determining the minimal calcium concentration which caused precipitation.

Five normal subjects were studied with each of the three cathartic/lavage solutions. Average age was 27 (range 23 to 36). Subjects were paid a fee for their participation. This research was approved by a review committee of Baylor University Medical Center, and informed consent was obtained.

### B. Study Protocol

Preparation time for the experiment and the experiment itself took a total of 36 hours. In the preparatory phase, the subjects did not consume any solid food after 12 midnight on day 1. From 7 a.m. until 7 p.m. the subjects were instructed to consume a clear liquid diet, to maintain good hydration, and to collect all of their urine in a jug that was provided to them. At 6:45 p.m. the subjects reported to the laboratory. At 7 p.m. they voided to complete the preliminary urine collection, entered the experimental period, and remained under our direct observation until the experiment was completed 17 hours later. At 7 p.m. they ingested or began to drink one of the solutions described below. Thereafter they collected all stool and urine in separate containers. At 5 a.m. on day 2, a blood sample was obtained and the subjects then ingested a second dose of their assigned solution. Urine and stool collection continued. At 8 a.m. another blood sample was obtained. At 12 noon, after final urine collection, the experiment ended.

Stools from 7 p.m. to 5 a.m. were pooled and diarrhea during this period was assumed to be due to the first dose of cathartic. Stools from 5 am to 12 noon were pooled and diarrhea during this period was assumed to be due to the second dose of cathartic. There were 3 separate urine collections: a) the 12 hour period preceding the first dose; b) from 7 pm to 5 am (10 hours); and c) from 5 am to 12 noon (7 hours). Throughout the experimental period blood pressure and pulse rate were measured at two hour intervals, in the lying position and after standing.

### C. Composition and Ingestion of Concentrated Sulfate Solution

Anhydrous sodium and potassium sulfate were purchased from Mallinckrodt Baker, Inc, Paris, Kentucky. Anhydrous magnesium sulfate was purchased from Fisher Scientific, Fairlawn, New Jersey. For a single dose, the weight and molar amount of the three salts were: Na₂SO₄, 17.51 grams (123.3 mmoles sulfate); K₂SO₄, 3.13 grams (18.0 mmoles sulfate); MgSO₄, 1.6 grams (13.3 mmoles sulfate). The amount of sulfate in two doses was 309 mmoles.

For each dose, the salts were dissolved in water, quantity sufficient to produce 100 mL of solution. This solution was swallowed, and followed immediately with 250 mL of water. During the next 3 hours, 1630 mL of additional water was ingested in divided doses at 30 minute intervals. Thus, with and after the ingestion of both doses of sulfate salts, subjects ingested 3960 mL of water. This included 200 mL of sulfate solution. Preliminary experiments (not shown) suggested that two doses of this sulfate solution would produce about 2600 grams of diarrheal stool. Therefore, the 3960 mL of ingested water exceeded expected stool water loss by about 1360 ml.

### D. Composition and Ingestion of Concentrated Phosphate Solution

Fleet^{®} Phospho-soda EZ-Prep bowel cleansing system (Fleet Laboratories, Lynchburg, Virginia) was used. According to the package insert, 15 mL of this solution contains 7.2 grams of monobasic sodium phosphate (NaH₂PO₄·H₂O) and 2.7 grams of dibasic sodium phosphate (Na₂HPO₄·7H₂O). Thus, each mL contains 128.6 mg of phosphorus (4.15 mmoles of phosphorus or phosphate). For the first dose, 45 mL of phospho-soda (containing 186.8 mmoles phosphate) was added to 360 mL of water. For the second dose, 30 mL of phospho-soda (124.5 mmoles phosphate) was added to 360 mL of water. The total dose of phosphate in both doses was 311 mmoles. After the first dose at 7 p.m., additional water was ingested at 7:30 p.m. (120 mL) and at half-hour intervals thereafter (250 mL each half hour) until 10 p.m. Thus in addition to 45 mL phospho-soda, the subjects received a total of 1730 mL of water after the first dose of phospho-soda. After the second dose additional water was ingested according to the following schedule: at 5 a.m. (120 mL), and at half hourly intervals thereafter (250 mL each half hour) until 7 a.m. Thus, in addition to 30 mL phospho-soda, the subjects received 1230 mL of water after the second dose. The total amount of water ingested with and after both doses of phosphate salts was 3035 mL. This includes 75 mL of phospho-soda solution. Expected stool water loss after 75 mL of phospho-soda was about 2000 mL [extrapolated from results of a previous study where a total of 90 mL phospho-soda was ingested (Patel, V, et al. (2007) Hum. Path., 38:193-194). Therefore, the 3035 mL of water ingested by these subjects exceeded their expected stool water loss by about 1035 mL.

### E. Composition and Ingestion of Peg Based Lavage Solution

The PEG lavage solution was Nulytely^{®} (Braintree Laboratories, Inc, Braintree, Massachusetts), which is dispensed in a jug containing PEG 3350, sodium chloride, sodium bicarbonate and potassium chloride. These ingredients were dissolved in water, quantity sufficient to produce 4 liters of solution. Two liters of lavage solution were ingested over a 2 hour period (250 mL every 15 minutes for 8 doses) starting at 7 p.m. on day 1. Two additional liters were ingested in the same manner, starting at 5 a.m. on day 2. Net water loss, or gain, as a direct result of the lavage solution was not anticipated, but additional water was administered to offset insensible losses according to the following schedule: 125 mL at 11 p.m., 1 a.m., 3 a.m., 9 a.m., and 11 a.m. This resulted in the ingestion of 625 mL of water over the 17 hour experimental period.

### E. Assessment of Diarrheal Fluid Cleansing

All diarrheal stool produced by a single bowel movement was well mixed by stirring and 14 mL were poured into a graduated conical centrifuge tube, which was capped and centrifuged for 30 minutes at 3000 rpm. The percent of insoluble fecal matter packed at the bottom of the tube was used as a measure of the degree to which diarrheal fluid had been cleansed.

### G. Chemical Analysis of Body Fluids

Serum concentrations of electrolytes and other plasma constituents were measured by automated analysis. Stool and urine solutes were analyzed in the Baylor GI Research Laboratory, as previously described (Fine, et al. (1998) Dig. Dis. Sci. 43:2708-2714). Phosphate concentration was measured by the colorimetric method of Fiske and Subbarow (Fiske, et al. (1925) J. Biol. Chem. 66:374-400), and expressed as mg of phosphorus or as mmoles of phosphorus or phosphate. Sulfate concentration was assayed by turbidity after addition of barium chloride. Percent water content of diarrheal stools was determined by weighing specimens before and after lyophilization.

Urine was also sent by overnight express to Litholink Corporation, Chicago, Illinois, for analysis of solutes which are believed to influence the development of renal stones (Asplin, et al. (1998) J. Urol. 159:1821-1825).

### H. [Ca] Concentration Induces Precipitation in Urine In Vitro

This experiment employed procedures previously described by Nicar, et al to assess the propensity for urine crystallization (Nicar, et al. (1983) Met. 32:906-910). Urine was collected with thymol. None of the specimens contained a precipitate by visual inspection and microscopy. Urine from the 12 hour preliminary period and urine collected for 7 hours after the second dose of cathartic/lavage solutions were studied.

Twenty-six 10 mL aliquots of urine were pipetted into test tubes and placed in a 37 °C water bath with constant stirring. In one set of 13 tubes the pH was maintained at 6.4 ± 0.1, with manual addition of 0.1 N HCI or NaOH, throughout a three-hour incubation period. In another set of 13 tubes pH was maintained at 7.2 ± 0.1. One tube at each pH served as controls. Increasing volumes of a concentrated solution of calcium chloride were added to the other tubes, progressively increasing the calcium concentration. The total volume of added solutions did not exceed 0.45 mL, thus avoiding dilution effects.

Precipitation after a three hour incubation period was identified visually by holding the tube in front of a light and by gentle agitation to reveal any precipitated material or aggregation of crystallization. The tube with the lowest calcium concentration which contained a precipitate was identified. The calcium concentration in that aliquot of urine was derived by adding the amount of calcium in 10 ml of native urine (as determined by atomic absorption spectroscopy) to the amount of calcium added to produce precipitation. The calcium concentration in the aliquot at the point of precipitation was then calculated in mg/dL.

### i. Statistical Analysis

One way analysis of variance (ANOVA) was used to test statistical significance of differences between the 3 treatment groups. When there were only two treatment groups to compare, such as phosphate absorption after ingestion of phosphate versus sulfate absorption after ingestion of sulfate, group t-test was used. When pre-treatment values and post-treatment values were available for each subject (such as blood pressure or urine values before and after a specific treatment), statistical significance of treatment effect was assessed by paired t-test. Correlation coefficients were calculated using Pearson Product Moment.

### II. Results

The intestine absorbed 35% of ingested phosphate and 17% of ingested sulfate, and stool volume was 2.0 L after phosphate, 2.9 L after sulfate, and 3.0 L after PEG. After phosphate ingestion, urine [PO₄] increased from 44 to 220 mg/dL; after sulfate ingestion, urine [SO₄] increased from 38 to 103 mg/dL. Calcium concentrations (in mg/dL) to induce urine precipitation at pH 6.4, before and after the cathartic regimens, were: PEG, from 26.6 to 42.5, p = 0.181; PO₄, from 28.2 to 7.4, p = <0.001; SO₄, from 31.2 to 211.1, p = 0.024.

### A. Blood Values and Vital Signs

The serum inorganic phosphate concentration was elevated after ingesting the phosphate solution: baseline, 1.05 ± 0.03 mmol/L (3.4 mg/dL); 10 hours after the first dose, 1.73 ± 0.03 mmol/L (5.6 mg/dL); and 3 hours after the second dose, 2.04 ± 0.22 mmol/L (6.6 mg/dL), p = 0.007. Corresponding mmol/L values for serum sulfate before and after ingestion of the sulfate solution were 0.31 ± 0.07, 0.60 ± 0.06, and 0.97 ± 0.05, p <0.001 . There were no other statistically significant changes or differences in measured serum chemical or hematologic parameters. Based on blood pressure and pulse rate, there was no statistically significant evidence of volume depletion in the 5 subjects who ingested any of the three cathartic/lavage solutions.

### B. Oral Intake and Stool Output During the 17 Hour Experimental Period

Because of the large molecular size of PEG 3350, 4 liters of PEG lavage solution contained on average only 3471 ml of water. Similarly, diarrheal fluid generated by PEG lavage contained less water per unit volume than diarrheal fluid produced by phosphate or sulfate. Therefore, stool volume and stool water are shown separately in Table 1.

**Table 1. Intake and stool output of water and solutes after ingestion of two doses of cathartics/lavage solutions plus additional water to mitigate dehydration**

| | PEG Lavage Solution (4L) 625 mL extra water | | | Concentrated Phosphate Solution (75 mL) 2960 mL extra water | | | Concentrated Sulfate Solution (200 mL) 3760 mL extra water | | | |
|---|---|---|---|---|---|---|---|---|---|---|
| | Intake | Stool output | Net* | Intake | Stool output | Net* | Intake | Stool output | Net* | P value ‡ |
| Volume, grams | 4625 | 3021±113 | - | 3035 | 2029±103 | -- | 3960 | 2911±220 | | 0.001 (output) |
| Water, grams | 4096 | 2564±113 | +1532±98 | 3035 | 1934±98 | +1101±98 | 3960 | 2801±213 | +1159±140 | 0.004 (output) |
| PEG, grams | 420 | 396±5 | +24±5 | -- | -- | -- | -- | -- | -- | -- |
| Phosphate, mmol | 0 | 5±0.7 | -5±0.7 | 311 | 203±16 | +108±16 | 0 | 8.4±1.6 | -8.4±1.6 | 0.004 (net) |
| Sulfate, mmol | 0 | 0.7±0.2 | -0.7±0.2 | 0 | 1.7±0.4 | 1.7±0.4 | 309 | 255±6 | +54±6 | 0.003 (net) |
| Magnesium, mmol | 0 | 5±1.2 | -5±1.2 | 0 | 8±2 | -8±2 | 25 | 31±2 | -5±2 | 0.302 (net) |
| Calcium, mmol | 0 | 3±0.4 | -3±0.4 | 0 | 3±0.3 | -3±0.3 | 0 | 10±2 | -10±2 | 0.007 (net) |
| Sodium, mmol | 260 | 153±22 | +107±22 | 363 | 291±18 | +72±18 | 489 | 476±53 | +12±54 | 0.179 (net) |
| Potassium, mmol | 20 | 27±3 | -7±3 | 0 | 44±5 | -44±5 | 71 | 60±7 | +11±6 | <0.001 (net) |
| Chloride, mmol | 212 | 83±14 | +129±14 | 0 | 14±6 | -14±6 | 0 | 48±17 | -48±17 | 0.004 (net) |
| Bicarbonate, mmol | 68 | 62±7 | +6±7 | 0 | 11±3 | -11±3 | 0 | 47±11 | -47±11 | 0.001 (net) |
| Stool pH 1^{st} dose | -- | 7.10±0.04 | -- | -- | 6.64±0.07 | -- | -- | 6.92±0.18 | -- | 0.114 (pH) |
| Stool pH 2^{nd} dose | -- | 8.00±0.12 | -- | -- | 6.80±0.07 | -- | -- | 7.50±0.13 | -- | <0.001 (pH) |

| | | | | | | | | | | |
|---|---|---|---|---|---|---|---|---|---|---|
| *Net absorption (denoted by positive signs) or net secretion (denoted by negative signs) ‡ p values by ANOVA | | | | | | | | | | |

Diarrheal stool volume was highest after PEG lavage, but the water output in stool was highest after sulfate. Sulfate catharsis produced 43 percent more stool volume than phosphate catharsis. Despite excreting large volumes of diarrheal fluid, subjects in all groups absorbed water during the 17 hour experimental period, averaging 1101 ml to 1532 ml.

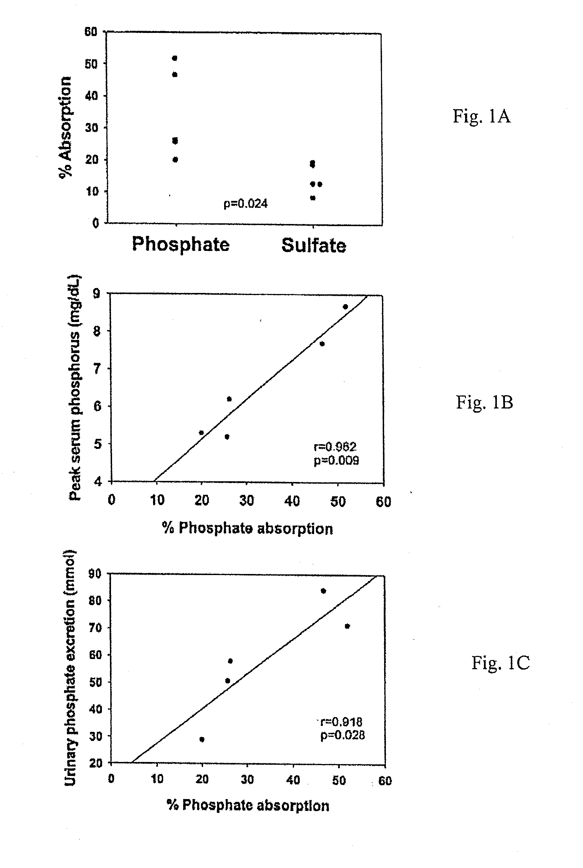

Net phosphate absorption after ingestion of the phosphate solution averaged 108 mmoles, approximately twice the average net sulfate absorption after ingestion of the sulfate solution (p = 0.014), as shown in Table 1 and Figure 1A. Among the 5 subjects who received the phosphate solution, the percent absorption of phosphate correlated with the 8 a.m. serum phosphate concentration (3 hours after the second dose of phosphate solution) and urine phosphate excretion (Figure 1B and 1C). Perhaps because there was relatively little variation in sulfate absorption after sulfate catharsis, no correlation was seen between sulfate absorption and serum sulfate concentration or urine sulfate excretion.

### C. Stool Weight and Cleansing of Diarrheal Fluid

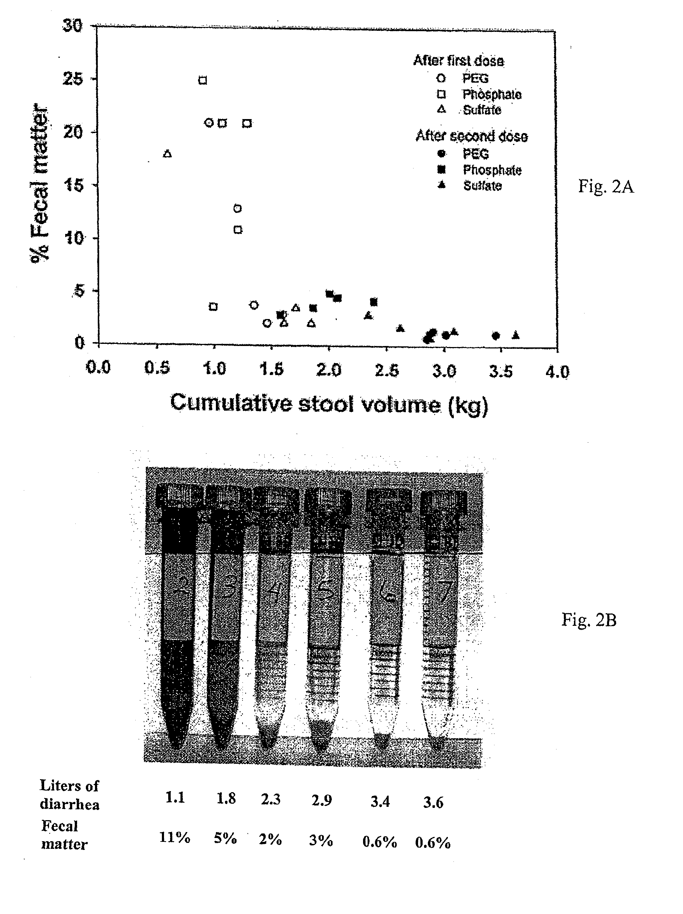

As shown in Figure 2A, increasing diarrheal stool weight was associated with a progressive reduction in percent insoluble fecal matter. After the first dose, average percent fecal matter in diarrheal fluid was 8.5 ± 3.7% with PEG, 6.4 ± 3.8% with sulfate, and 16.4 ± 4.0% with phosphate (p= 0.203). After the second dose, average percent fecal matter in diarrheal fluid was 1.1 ± 0.1% with PEG, 1.6 ± 0.4% with sulfate, and 4.1 ± 0.4% with phosphate (p = <0.001). Figure 2B shows the appearance of centrifuged diarrheal stool at different stages of diarrheal fluid cleansing, for comparison with results shown in 2A.

### D. Urine Volume and Urine Output of Solutes

Average urine volume during the preliminary 12 hour period for all 15 studies was 1798 ± 230 mL. Average urine volume during the 17 hour experimental period ranged from 1624 to 2006 ml (Table 2).

**Table 2. Volume and solute output in urine during the 17 hour experimental period.**

| | PEG (n=5) | PO₄ (n=5) | SO₄ (n=5) | p |
|---|---|---|---|---|
| Volume, grams | 1624±159 | 1678±79 | 2006±179 | 0.176 |
| Urine PO₄, mmol | 16±1 | 75±10 | 24±5 | <0.001 |
| Urine SO₄, mmol | 8±1 | 10±1 | 49±2 | <0.001 |
| Urine Ca, mmol | 2.4±0.5 | 0.9±0.1 | 2.6±0.3 | 0.009 |
| Urine Mg, mmol | 2.3±0.2 | 1.5±0.3 | 3.7±0.5 | 0.005 |
| Urine Na, mmol | 100±30 | 103±17 | 133±33 | 0.671 |
| Urine K, mmol | 28±4 | 38±6 | 46±8 | 0.161 |
| Urine Citrate, mmol | 0.7±0.2 | 0.4±0.1 | 1.2±0.4 | 0.171 |
| Urine Oxalate, mmol | 0.2±0.01 | 0.4±0.09 | 0.2±0.02 | 0.056 |
| Urine Net Acid, mEq | 33±4 | 90±8 | 61±7 | <0.001 |

| | | | | |
|---|---|---|---|---|
| Citrate and oxalate measurements shown here were made by Litholink Corporation. All other measurements were made in the Baylor G1 Research Laboratory. | | | | |

As shown earlier in Table 1, ingestion of 311 mmoles of phosphate resulted in net absorption of 108 mmoles of phosphate. Seventy five mmoles of phosphate were excreted in urine after phosphate ingestion (Table 2). Since 16 mmoles of phosphate were excreted in urine after ingestion of PEG lavage solution (which contains no phosphate), an estimate of 59 of 108 mmoles of absorbed phosphate (55%) was excreted in the urine during the 17 hour urine collection period. By similar calculations, following sulfate ingestion, 76% of the absorbed sulfate (41 of 54 mmoles) was excreted in the urine during the 17 hour urine collection period.

Urine calcium excretion was similar after ingestion of sulfate and PEG solutions, but was substantially reduced after ingestion of phosphate (Table 2). Urine magnesium excretion increased after ingestion of sulfate. Urine net acid secretion was higher after phosphate and sulfate than after PEG.

### E. Relative Saturation Ratios for Brushite (RSR-Br) in Urine

The data in Table 3 were measured by Litholink Corporation, who also analyzed the results with the EQUIL2 interactive computer program to calculate RSR-Br (Asplin, et al. (1997), Kidney Int. 52:1602-1608; Werness, et al. (1985) J. Urol. 134:1242-1244). Brushite is the initial calcium phosphate precipitate which develops in biologic solutions such as urine or renal tubular fluid. It has a molar Ca:P ratio of 1.0. This precipitate may be converted to other forms of calcium phosphate which have a higher Ca:P ratio, but the BSR for brushite is of principle interest in regard to precipitation of calcium phosphate in biologic fluids (Pak (1969) J. Clin. Invest. 1914-1922).

| Table 3. Solute concentrations in urine, and calculation of relative saturation ratio for brushite (RSR-Br) and creatinine clearance. | | | | | | | | | |
|---|---|---|---|---|---|---|---|---|---|
| | Preliminary period | | | After 1^{st} dose (7 p.m. - 5 a.m.) | | | After 2^{nd} dose (5 a.m. - 12 p.m.) | | |
| | PEG | PO₄ | SO₄ | PEG | PO₄ | SO₄ | PEG | PO₄ | SO₄ |
| Urine flow, mL/h | 125±32 | 175±37 | 144±31 | 106±12 | 114±12 | 116±17 | 80±18 | 79±19 | 119±32 |
| Na, mEq/L | 65±14 | 94±23 | 87±22 | 51±12 | 65±7 | 96±28 | 76±14 | 64±9 | 59±11 |
| K, mEq/L | 43±13 | 34±10 | 39±6 | 13±3 | 23±3 | 29±6 | 26±5 | 34±11 | 27±5 |
| Cl, mEq/L | 70±14 | 91±23 | 81±21 | 41±11 | 34±5 | 68±26 | 84±17 | 24±6 | 28±8 |
| Creatinine, mg/dL | 110±31 | 77±22 | 115±38 | 73±13 | 81±5 | 90±15 | 123±24 | 141±37 | 96±20 |
| Mg, mg/dL | 4.5±1.1 | 4.2±1.7 | 5.0±1.3 | 2.6±0.5 | 2.1±0.5 | 4.0±1.1 | 6.1±1.0 | 2.9±0.8 | 6.8±1.4 |
| Ca, mg/dL | 6.6±2.0 | 6.7±1.9 | 8.0±2.7 | 3.9±1.0 | 1.9±0.3 | 4.7±0.9 | 10±2 | 2.4±0.8 | 7.3±1.8 |
| P, mg/dL | 41±12 | 44±14 | 72±32 | 30±5 | 127±24 | 45±8 | 35±9 | 220±36 | 41±13 |
| SO₄, mEq/L | 17±5 | 15±5 | 24±9 | 10±2 | 11±1 | 40±9 | 14±3 | 16±4 | 64±14 |
| Citrate, mg/dL | 13±4 | 10±4 | 13±4 | 6.7±1.6 | 4.1±0.7 | 14±6 | 13±3 | 7.8±4.7 | 10±2 |
| NH₄, mEq/L | 20±5 | 15±5 | 28±12 | 15±4 | 19±4 | 20±3 | 24±5 | 32±4 | 28±7 |
| Oxalate, mg/dL | 1.6±0.5 | 1.2±0.2 | 1.9±0.7 | 1.0±0.2 | 2.0±0.7 | 1.2±0.2 | 1.4±0.3 | 4.2±2.5 | 1.1±0.2 |
| UA, mg/dL | 36±7 | 29±7 | 33±14 | 20±2 | 20±2 | 28±5 | 24±2 | 24±5 | 19±6 |
| UUN, mg/dL | 533±90 | 510±150 | 679±209 | 331±52 | 375±37 | 456±58 | 482±77 | 501±69 | 430±74 |
| pH | 6.48±0.25 | 6.67±0.11 | 6.31±0.30 | 6.29±0.23 | 5.87±0.17 | 6.15±0.26 | 6.16±0.35 | 5.51±0.06 | 5.59±0.16 |
| **RSR-Br** | **0.80±0.22** | **1.18±0.40** | **1.11±0.39** | **0**.**49**±**0**.**16** | **0.31±0.07** | **0.48±0.18** | **0.67±0.18** | **0.25±0.02** | **0.23±0.10** |
| **Creatinine Clearance** | | | | | | | | | |
| ml/min/1.73M² | 186±36 | 158±6.6 | 168±18 | 125±12 | 134±8.9 | 131±16 | 142±5.9 | 125±11 | 136±12 |

Urine collected during the preliminary period had RSR-Br values close to 1.0, indicating saturated or near saturated conditions with respect to brushite (Table 3). Compared to the preliminary urine, the RSR-Br of urine collected after all three cathartic/lavage solutions fell substantially below 1. For example, the RSR-Br after phosphate ingestion fell from 1.18 to 0.25 despite a sharp rise in urine phosphate concentration. This occurred because of the marked reduction in urine calcium concentration and the fall in urine pH.

### F. Creatinine Clearance

Compared to values obtained in the 12 hour preliminary period, average creatinine clearance fell slightly after all cathartic/lavage solutions (Table 3), but these changes were not statistically significant. Creatinine clearance after the first and second dose of phosphate and sulfate were similar to clearances after the PEG solution.

### G. [Ca] Induces Precipitation in Urine In Vitro at pH 6.4 and 7.2

Table 4 compares urine from the baseline period to urine collected following the second dose of cathartic/lavage solutions.

**Table 4. Effect of three cathartic/lavage regimens on the calcium concentration required to induce precipitation in urine at pH 6.4 and 7.2. Concentrations are expressed in mg/dL, mean ± SEM.**

| | PEG | **pH 6.4** PO₄ | SO₄ | PEG | **pH 7.2** PO₄ | SO₄ |
|---|---|---|---|---|---|---|
| Before cathartic | **26.6** ±4.5 | **28.2** ±1.5 | **31.2** ±7.3 | **10.1** ±1.1 | **14.2** ±3.0 | **15.7** ±2.9 |
| After second dose of cathartic | **42.5** ±12.0 | **7.4** ±0.6 | **211.1** ±56.9 | **12.6** ±1.4 | **5.6** ±1.0 | **35.9** ±12.7 |
| *p* value* | **0.181** | **<0.001** | **0.024** | **0.131** | **0.032** | **0.218** |

| | | | | | | |
|---|---|---|---|---|---|---|
| * p values shown here were determined by paired t-test, comparing results on urine specimens from each subject before and after cathartic/lavage solutions were ingested. In a separate analysis by ANOVA, results on the three groups of subjects who were to later receive different cathartic/lavage solutions were not significantly different; after the second dose of cathartic/lavage solutions p values for differences were 0.003 at pH 6.4 and 0.004 at pH 7.2. | | | | | | |

At pH 6.4 PEG did not change the calcium concentration to produce urine precipitation. In contrast, phosphate ingestion markedly reduced the calcium concentration to evoke precipitation, while sulfate markedly increased the calcium concentration to induce precipitation.

Calcium phosphate precipitation occurs much more readily at higher than at lower pH values, and thus it is not surprising that at pH 7.2 much lower calcium concentrations generated a precipitate compared with results at pH 6.4. This may explain why at pH 7.2 sulfate did not significantly increase the calcium concentration to induce precipitation. On the other hand, phosphate caused a statistically significant decrease in the calcium concentration to evoke precipitation at pH 7.2 (Table 4), as it did at pH 6.4.

The quantity of precipitate that developed after addition of calcium was sufficient for further analysis in 44 of 60 experiments conducted at either pH 6.4 or 7.2. The dried precipitates were shipped to Mayo Medical Laboratories (Rochester, MN) for analysis by infrared spectroscopy. Forty three of 44 precipitates contained calcium phosphate. Usually this was the only precipitate; in some instances small amounts of calcium oxalate or a magnesium containing salt were also found. The final calcium phosphate salt which was found at pH 7.2 was primarily calcium hydroxy apatite while both calcium hydroxy apatite and brushite were seen at pH 6.4. The precipitate which formed in the urine of one of the five subjects after ingestion of the sulfate solution was calcium sulfate at pH 6.4 (calcium concentration at precipitation was 32 mg/dL); at pH 7.2, the precipitate in this urine was calcium phosphate (calcium concentration at precipitation was 12 mg/dL).

### III. Conclusions

1) In equimolar doses, sulfate is a more effective cathartic than phosphate. 2) Phosphate catharsis increases the propensity for calcium salt precipitation in urine at pH 6.4 (the pH of distal tubular fluid), whereas sulfate catharsis decreases the likelihood of such precipitation. 3) These observations suggest that sulfate would be less likely than phosphate to induce calcium salt deposition in renal tubular fluid.

### EQUIVALENTS

Those skilled in the art will recognize, or be able to ascertain, using no more than routine experimentation, numerous equivalents to the specific compositions and procedures described herein. Such equivalents are considered to be within the scope of this invention, and are covered by the following claims.

Disclosed are the following items:
1. A method of treating a hypercalcium concentration-related disorder in a patient in need thereof, the method comprising administering a therapeutic composition comprising sodium sulfate, potassium sulfate, and magnesium sulfate.
2. The method of item 1, wherein the therapeutic composition is in the form of a solution.
3. The method of item 1, wherein the disorder is selected from the group consisting of hypercalciuria, nephrocalcinosis, recurring calcium nephrolithiasis, calcium phosphate stone disease, stone forming disease, struvite formation, calcium-phosphate recurrent stones, absorptive hypercalciuria syndrome, vascular calcification, recurrent calcium urolithiasis, persistently high urinary pH, calciphylaxis, calciphalaxis, chronic renal failure, end stage renal disease, calcific uremic arteriolopathy
4. A method of reducing the risk of renal calcinosis occurring from the ingestion of a lavage solution in a patient with a propensity for renal stones, the method comprising administering a therapeutically effective amount of a lavage solution consisting of sodium sulfate, potassium sulfate, and magnesium sulfate in the patient.
5. The method of item 4, wherein the lavage solution contains between about 5 g to about 30 g of sodium sulfate, between about 1 g to about 5 g potassium sulfate, and between about 0.5 g to about 5 g of magnesium sulfate.
6. The method of item 4, wherein the volume of the lavage solution is between about 75 ml and 125 ml.
7. The method of item 4, wherein there is a first administration of the lavage solution and a second administration of the lavage solution.
8. The method of item 5, wherein the lavage solution contains about 17 g of sodium sulfate, about 3 grams of potassium sulfate, and about 1.5 g of magnesium sulfate.
9. The method of item 1, wherein the administration of the lavage solution is immediately followed by the administration of water.
10. The method of item 9, wherein the volume of the water administered immediately following the lavage solution is between about 200 ml and 300 ml.
11. The method of item 10, further comprising an additional administration of water.
12. The method of item 11, wherein the volume of water administered is between about 1200 ml and about 2000 ml.
13. The method of item 12, wherein the volume of water administered is about 1600 ml.
14. The method of item 11, wherein the additional administration of water after the administration of the lavage solution is divided into equal doses administered every 30 minutes for three hours.
15. The use of sodium sulfate, potassium sulfate, and magnesium sulfate in the manufacture of a medicament for reducing the risk of renal calcinosis occurring from the ingestion of a lavage solution in a patient with a propensity for renal stones, comprising administering to the patient a therapeutic comprising sodium sulfate, potassium sulfate, and magnesium sulfate in the patient.
16. The use of sodium sulfate, potassium sulfate, and magnesium sulfate in the manufacture of a medicament for the treatment a hypercalcium concentration-related disorder in a patient in need thereof.

## Claims

1. A composition in the form of a solution, the composition comprising sodium sulfate, potassium sulfate, and magnesium sulfate for use in the prevention of a hypercalcium concentration-related disorder in a patient.

2. The composition for the use according to claim 1, wherein the prevention of a hypercalcium-related disorder comprises alleviating symptoms associated with said disorder, the inhibition of the progression of said disorder, and the promotion of the regression of said disorder.

3. The composition for use according to claim 1, wherein the disorder is selected from the group consisting of hypercalciuria, nephrocalcinosis, recurring calcium nephrolithiasis, calcium phosphate stone disease, stone forming disease, struvite formation, calcium-phosphate recurrent stones, absorptive hypercalciuria syndrome, vascular calcification, recurrent calcium urolithiasis, persistently high urinary pH, calciphylaxis, chronic renal failure, end stage renal disease, calcific uremic arteriolopathy.

4. The composition for use according to claim 1 or 2, wherein the dose of total sulfate derived magnesium sulfate, sodium sulfate, and potassium sulfate is between 0.01 mg/ml to 100 mg/ml, or 0.1 mg/ml to 10 mg/ml, or 1 mg/ml to 5 mg/ml.

5. The composition for use according to any one of claims 1-3, wherein the composition is formulated for administration comprising oral, anal, systemic, or localized administration.

6. The composition according to any of the preceding claims, wherein the composition is administered in combination with another preventative treatment for a hypercalcium concentration-related disorder.

7. The composition according to claim 7, wherein the other preventative treatment is administered prior to, subsequent to, or concomitantly with the composition according to any of the preceding claims.
